(19) Europäisches Patentamt
European Patent Office
Office européen des brevets

(11) Publication number: 0 318 262 A1

(12) EUROPEAN PATENT APPLICATION

(21) Application number: 88311076.9

(51) Int. Cl.⁴: C10L 1/22

(22) Date of filing: 23.11.88

(30) Priority: 24.11.87 GB 8727504

(43) Date of publication of application: 31.05.89 Bulletin 89/22

(84) Designated Contracting States: AT BE CH DE ES FR GB GR IT LI NL SE

(71) Applicant: GLAXO GROUP LIMITED
Clarges House 6-12 Clarges Street
London W1Y 8DH(GB)

(72) Inventor: Payne, Henry Arthur Sheldon
17 Deanburn Road Linlithgow
West Lothian Scotland(GB)
Inventor: Ogden, Helen Isabella Grace
6 Ravelsykes Road Cornbank Penicuik
Midlothian Scotland(GB)

(74) Representative: Holmes, Michael John et al
Frank B. Dehn & Co. Imperial House 15-19
Kingsway
London WC2B 6UZ(GB)

(54) **Compositions.**

(57) Compounds of formula

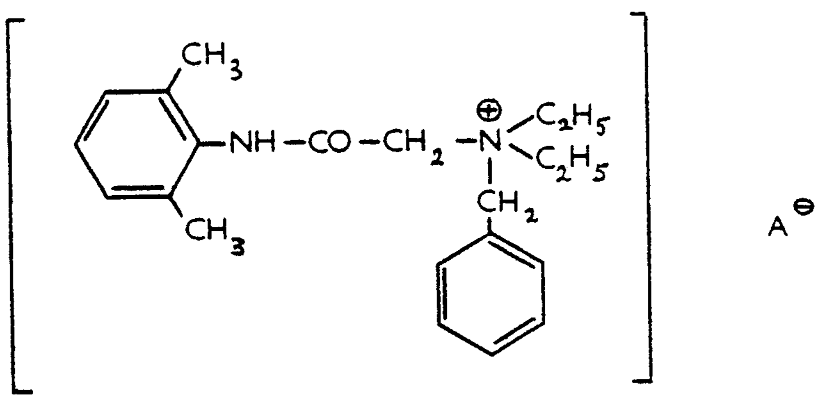

wherein $A^{\ominus}$ is an anion, can be used as aversive agents for apolar hydrocarbon-based liquids. The low solubility of the compounds in apolar liquids may be overcome by addition of a surfactant without deleteriously affecting the bittering properties of the compounds.

EP 0 318 262 A1

## COMPOSITIONS

This invention relates to the use of lignocaine benzyl benzoate and related substances as aversive agents for liquid hydrocarbons.

Liquid hydrocarbons are widely used and kerosene and white spirit are to be found in most households. The incidence of domestic accidents arising from inadvertent drinking of these liquids is very high and their physiological effects are especially damaging, and may often be fatal. Moreover, in third world countries, near starving children will frequently drink almost any available liquid, including kerosene which is widely used as a fuel. It is thus desirable to find an aversive agent suitable for use in such hydrocarbon liquids.

The substance lignocaine benzyl benzoate (denatonium benzoate; sold under the Trade Mark Bitrex) has been said to be the bitterest substance known to man (Guinness Book of Records). It is described in British Patent Specification No. 955309 together with related aversive agents of similar structure. Lignocaine benzyl benzoate has been used, for example, for denaturing methylated spirits, and more recently is being used as an aversive agent in a number of household products such as disinfectant and floor cleaner. Although such products naturally have an unpleasant taste, this may not be enough to deter children and the extreme bitterness of lignocaine benzyl benzoate, even at concentrations as low as 10 parts per million (ppm), ensures that their reaction is to stop drinking the product immediately before significant swallowing has taken place.

It has been proposed speculatively to use lignocaine benzyl benzoate as an aversion agent for kerosene and white spirit (Pearson, The Recorder, 3 July 1986, published by Recorder Newspapers, 359 High Road, Ilford, Essex, England) but there is a problem with such a formulation in that lignocaine benzyl benzoate and related aversion agents are extremely insoluble in such apolar liquids as hydrocarbons. In the past, lignocaine benzyl benzoate has only been included in polar solutions or in the form of suspensions or dispersions where this problem of lack of solubility did not arise. Even though lignocaine benzyl benzoate is effective at concentrations as low as 10ppm, it is found that it is not possible to dissolve sufficient of this substance in kerosene to form a stable solution having the required level of bitterness. Furthermore, the tiny amount which does dissolve, rapidly migrates to the container walls.

Theoretically, it might be possible to form a lipophilic derivative or analogue of lignocaine benzyl benzoate which would be soluble in hydrocarbons. However, it appears that an important requirement of an aversive agent for preventing ingestion is that the agent dissolves very rapidly in the saliva of the mouth in order to produce the aversive reaction almost immediately. Thus, it is important to maintain the high water solubility of the lignocaine benzyl quaternary salt, and its low lipophilicity is thus an essential property.

Our experiments have shown that it is possible to produce stable solutions of a lignocaine benzyl quaternary salt in a liquid hydrocarbon at suitable concentrations by incorporation of a surfactant, and that on tasting the extreme aversive reaction is as rapid as in other products in which the agent has been used.

According to the present invention, therefore, we provide apolar hydrocarbon-based liquids containing an effective amount of a compound of the general formula

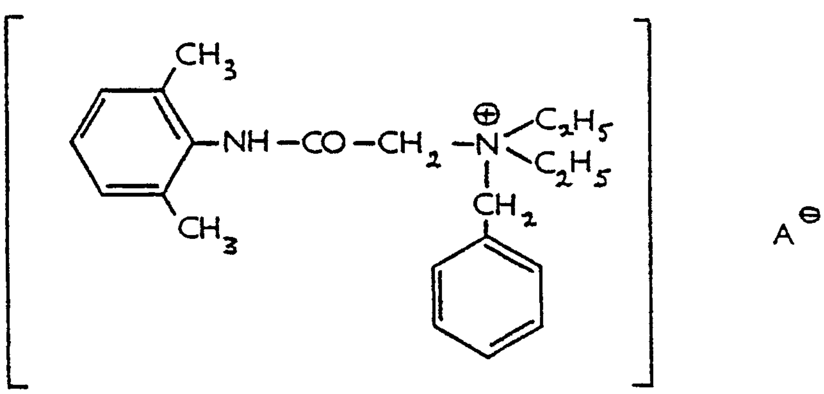

wherein $A^{\ominus}$ is an anion, as an aversive agent, together with a surfactant whereby a stable solution or suspension of said agent is formed.

The aversive agent is a lignocaine benzyl quaternary salt, preferably the benzoate. Other possible salts include inorganic acid salts e.g. halides such as the chloride; carboxylate salts such as the stearate;

sulphonic acid salts. such as the cyclamate and other salts such as the saccharide.

The concentration of aversive agent in the liquid will depend on the nature of any other substances which may be present and the intended destination of the product. Thus, for use in third world countries a high concentration may be necessary to deter starving children. On the other hand, with products used domestically in bulk, cost is an important factor leading to choice of lower concentrations of the aversive agent. In general the concentration of aversive agent will be in the range 1 to 5000ppm, for example 1 to 1000ppm, preferably 5 to 100ppm and more preferably 10 to 50ppm.

The surfactant may be a non-ionic, anionic or cationic surfactant. In general, ionic surfactants are preferred but surprisingly, cationic surfactants have so far given the best results with lignocaine benzyl benzoate although it had been expected that the cationic lignocaine benzyl ion would have no affinity for a further cationic molecule.

Suitable cationic surfactants include hydrocarbon-soluble quaternary ammonium surfactants (for instance tricaprylmethylammonium, cetylpyridinium and tri-($C_{8-10}$)-alkylmethylammonium chlorides), high molecular weight fatty amine surfactants, primary, secondary and tertiary amine surfactants, imidazoline surfactants, ether amine surfactants, alkyl propylene diamine surfactants and quaternary ester surfactants.

Non-ionic surfactants include hydrocarbon-soluble alkylphenol ethoxylate surfactants, alcohol ethoxylate surfactants (for instance polyethylene glycol hexadecyl and 4-tert-octylphenyl ethers), amine and ester ethoxylate surfactants, castor oil and acid ethoxylate surfactants, alkylolamide surfactants, propoxylate surfactants, block copolymer surfactants and ester surfactants.

Anionic surfactants include hydrocarbon-soluble alkylaryl sulphonate surfactants (for instance amine salts of alkyl benzene sulphonic acids), alcohol sulphate surfactants (for instance sodium 2-ethylhexyl sulphate and sodium dodecyl sulphate), ether sulphate surfactants, phosphate ester surfactants, sulphasuccinate surfactants, alkane, olefin and petroleum sulphonate surfactants, sarcosinate surfactants, taurate/isothionate surfactants and long-chain fatty acid salt surfactants.

Miscellaneous other surfactants include amine oxide surfactants, fluorinated compound surfactants, glycoside surfactants, lanolin and other woolwax derivatives, protein derivative surfactants and sequestering agents.

Mixtures of surfactants may also be used, for instance mixtures of quaternary ammonium surfactants such as tricaprylmethylammonium chloride with other surfactants.

The concentration of the surfactant will in part depend on the nature of the material but in general will be 20 to 100 times that of the aversive agent i.e. in the range from 20ppm, and preferably from 50 ppm, up to about 5% by weight. It is, of course, important that the function of the liquid hydrocarbon product is not significantly affected, for example the inflammability of kerosene or the solvent characteristics of white spirit. In general, lower concentrations are preferred, for example 200 to 10,000 ppm and preferably 1000ppm to 10,000ppm, for instance about 2000 ppm.

The hydrocarbon based liquid may, for example, be kerosene (paraffin), white spirit or other hydrocarbon solvents, petrol, benzene or cleaning fluid. Other examples of hydrocarbon-based liquids which may be treated according to the invention are kerosene-like hydrocarbon mixtures, such as petroleum distillate, deobase (purified kerosene), diesel oil, fuel oil and gasoline (petrol); varnish makers' and painters' naphthas (VM & P naphthas, or mineral spirits); crude petroleum; liquid petrolatum; and turpentine. For definitions of these common terms reference may be made to, for instance, the textbook "Clinical Toxicology of Commercial Products" by R.E Gosselin et al, 4th edition published 1976 by William and Wilkins, Baltimore, USA.

Other substances may be present, for example dyestuffs to provide distinctive colours.

Our tests have shown that lignocaine benzyl benzoate solutions in hydrocarbon liquids containing appropriate levels of surfactant are stable for at least one year at room temperature.

A further advantage is that where higher levels of surfactants are used, e.g. 1% by weight, the hydrocarbon liquid can be removed by water washing from, for example, the hands or containers. On the other hand, the performance of the hydrocarbon liquid is not deleteriously affected.

The hydrocarbon solutions may conveniently be produced by dissolution in the basic hydrocarbon of a pre-formed mixture of the aversive agent and the surfactant. Such preferred mixtures are new and constitute a further feature of the invention.

Thus according to a further feature of the invention we provide a composition comprising a compound of the general formula

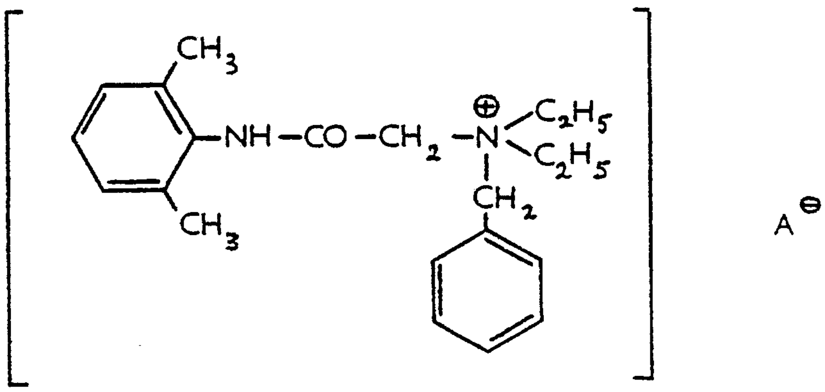

wherein $A^{\ominus}$ is an anion, and a surfactant, said composition forming a stable solution or suspension when dispersed in an apolar hydrocarbon-based liquid. The ratio of surfactant to aversive agent in the pre-formed concentrates according to the invention will be such as to provide the desired final concentrations in the hydrocarbon liquid, when dispersed or dissolved.

The aversive agent is conveniently present in the preformed concentrate at a concentration of 0.5 to 5.0% by weight, preferably 0.5 to 2.0% by weight. The concentration of surfactant is conveniently 10 to 99.5% by weight, preferably 30 to 99.5% by weight. Any remainder may comprise the hydrocarbon in which the concentrate is ultimately to be dispersed, or any other suitable apolar liquid.

According to a further aspect of the invention there is provided a method of rendering an apolar hydrocarbon-based liquid unpalatable, comprising the addition to said liquid of an effective amount of an aversive agent of the general formula.

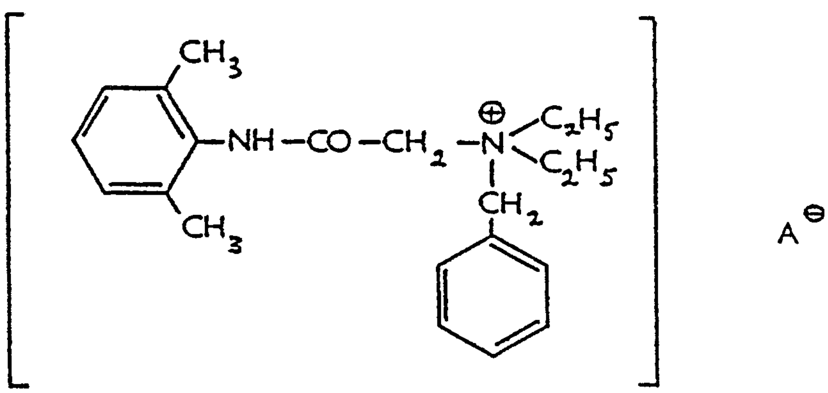

wherein $A^{\ominus}$ is an anion, together with a surfactant, whereby a stable solution or suspension of the said agent is formed. Preferably the method comprises adding a pre-formed mixture of surfactant and aversive agent to the hydrocarbon-based liquid, as described above, although simple dissolution of the aversive agent in the liquid and surfactant is also possible.

The invention is illustrated by the following Examples. Percentages are by weight throughout. Aliquat 336 and Alimod 2 are tricaprylmethylammonium chloride surfactants available from Henkel Chemicals, Enfield, Middlesex, England. Adogen 464 is a tri-($C_{8-10}$)-alkylmethylammonium chloride surfactant available from Ashland Chemicals Co., Columbus, Ohio, USA. Synperonic NP4 and 91/2.5 are alkylphenol ethoxylate and primary alcohol ethoxylate surfactants respectively, available from ICI, Wilton, Middlesbrough, Cleveland. Pentrone A4D is an amine salt of an alkyl benzene sulphonic acid available from ABM Chemicals Ltd, of Woodley, Stockport, Cheshire, England. Brij 52 is a polyethylene glycol 4-tert-octylphenol ether available from Atlas Chemical Industries (UK) Limited of Leatherhead, Surrey, England.

EXAMPLE 1

100 ppm Lignocaine benzyl benzoate/1% Aliquat 336

A 15% standard solution of lignocaine benzyl benzoate in methanol was prepared. 0.1 ml was pipetted into a 150 ml clear, flat, bottle, and the methanol allowed to evaporate.

A 5% solution of the surfactant in white spirit was prepared and 30 ml transferred to the test bottle. The surfactant solution and lignocaine benzyl benzoate were well mixed and 120 ml white spirit added to bring the total volume to 150 ml. This solution was then shaken vigorously.

EXAMPLE 2

50 ppm Lignocaine benzyl benzoate/1% Adogen 464

A 7.5% standard solution of lignocaine benzyl benzoate in methanol was prepared. 0.1ml was pipetted into a 150 ml clear, flat bottle, and the methanol allowed to evaporate.

A 5% solution of the surfactant in white spirit was prepared and 30ml transferred to the test bottle. The surfactant solution and lignocaine benzyl benzoate were well mixed and 120 ml white spirit added to bring the total volume to 150 ml. This solution was then shaken vigorously.

EXAMPLE 3

100 ppm Lignocaine benzyl benzoate/1% Synperonic 91/2.5

A 15% standard solution of lignocaine benzyl benzoate in methanol was prepared. 0.1 ml was pipetted into a 150 ml clear, flat bottle, and the methanol allowed to evaporate.

A 5% solution of the surfactant in white spirit was prepared and 30 ml transferred to the test bottle. The surfactant solution and lignocaine benzyl benzoate were well mixed and 120 ml white spirit added to bring the total volume to 150 ml. This solution was then shaken vigorously.

EXAMPLE 4

100 ppm Lignocaine benzyl benzoate/1% Synperonic NP4

A 15% standard solution of lignocaine benzyl benzoate in methanol was prepared. 0.1 ml was pipetted into a 150 ml clear, flat bottle, and the methanol allowed to evaporate.

A 5% solution of the surfactant in white spirit was prepared and 30 ml transferred to the test bottle. The surfactant solution and lignocaine benzyl benzoate were well mixed and 120 ml white spirit added to bring the total volume to 150 ml. This solution was then shaken vigorously.

EXAMPLE 5

50 ppm Lignocaine benzyl benzoate/0.5% Pentrone A4D

A 7.5% standard solution of lignocaine benzyl benzoate in methanol was prepared. 0.1ml was pipetted into a 150ml clear, flat botle, and the methanol allowed to evaporate. A 5% standard solution of the surfactant in white spirit was prepared and 15ml transfered to the test bottle. The surfactant and lignocaine benzyl benzoate were well mixed and 135ml white spirit added to bring the total volume to 150ml. This solution was then shaken vigorously.

EXAMPLE 6

50 ppm Lignocaine benzyl benzoate/1.0% Pentrone A4D

A 7.5% standard solution of lignocaine benzyl benzoate in methanol was prepared. 0.1ml was pipetted into a 150ml clear, flat botle, and the methanol allowed to evaporate. A 5% standard solution of the surfactant in white spirit was prepared and 30ml transfered to the test bottle. The surfactant and lignocaine benzyl benzoate were well mixed and 120ml white spirit added to bring the total volume to 150ml. This solution was then shaken vigorously.

EXAMPLE 7

50 ppm Lignocaine benzyl benzoate/0.5% Aliquat 336 in Paraffin

A 7.5% standard solution of lignocaine benzyl benzoate in methanol was prepared. 0.1ml was pipetted into a 150ml clear, flat botle, and the methanol allowed to evaporate. A 5% standard solution of the surfactant in paraffin was prepared and 15ml transfered to the test bottle. The surfactant and lignocaine benzyl benzoate were well mixed and 135ml paraffin added to bring the total volume to 150ml. This solution was then shaken vigorously.

EXAMPLE 8

50 ppm Lignocaine benzyl benzoate/0.5% Brij 52 in Paraffin

A 7.5% standard solution of lignocaine benzyl benzoate in methanol was prepared. 0.1ml was pipetted into a 150ml clear, flat bottle, and the methanol allowed to evaporate. A 5% standard solution of the surfactant in paraffin was prepared and 15ml transferred to the test bottle. The surfactant and lignocaine benzyl benzoate were well mixed and 135ml paraffin added to bring the total volume to 150ml. This solution was then shaken vigorously.

EXAMPLE 9

50 ppm Lignocaine benzyl cyclamate/0.5% Aliquat 336

A 7.5% standard solution of lignocaine benzyl cyclamate in methanol was prepared. 0.1ml was pipetted into a 150ml clear, flat bottle, and the methanol allowed to evaporate. A 5% standard solution of the surfactant in white spirit was prepared and 15ml transferred to the test bottle. The surfactant and lignocaine benzyl cyclamate were well mixed and 135ml white spirit added to bring the total volume to 150ml. This solution was then shaken vigorously.

EXAMPLE 10

100 ppm Lignocaine benzyl stearate/1.0% Aliquat 336

A 15% standard solution of lignocaine benzyl stearate in methanol was prepared. 0.1ml was pipetted into a 150ml clear, flat bottle, and the methanol allowed to evaporate. A 5% standard solution of the surfactant in white spirit was prepared and 30ml transferred to the test bottle. The surfactant and lignocaine benzyl stearate were well mixed and 120ml white spirit added to bring the total volume to 150ml. This solution was then shaken vigorously.

EXAMPLE 11

30 ppm Lignocaine benzyl benzoate/0.15% Aliquat 336

A 4.5% standard solution of lignocaine benzyl benzoate in methanol was prepared. 0.1ml was pipetted into a 150ml clear, flat bottle, and the methanol allowed to evaporate. A 5% standard solution of the surfactant in white spirit was prepared and 4.5ml transferred to the test bottle. The surfactant and lignocaine benzyl benzoate were well mixed and 145.5ml white spirit added to bring the total volume to 150ml. This solution was then shaken vigorously:

EXAMPLE 12

40 ppm Lignocaine benzyl benzoate/0.4% Aliquat 336

A 6% standard solution of lignocaine benzyl benzoate in methanol was prepared. 0.1ml was pipetted into a 150ml clear, flat bottle, and the methanol allowed to evaporate. A 5% standard solution of the surfactant in white spirit was prepared and 12ml transferred to the test bottle. The surfactant and lignocaine benzyl benzoate were well mixed and 138ml white spirit added to bring the total volume to 150ml. This solution was then shaken vigorously.

EXAMPLE 13

40 ppm Lignocaine benzyl benzoate/0.08% Aliquat 336

A 6% standard solution of lignocaine benzyl benzoate in methanol was prepared. 0.1ml was pipetted into a 150ml clear, flat bottle, and the methanol allowed to evaporate. A 5% standard solution of the surfactant in white spirit was prepared and 2.4ml transferred to the test bottle. The surfactant and lignocaine benzyl benzoate were well mixed and 147.6ml white spirit added to bring the total volume to 150ml. This solution was then shaken vigorously.

EXAMPLE 14

80 ppm Lignocaine benzyl benzoate/0.8% Aliquat 336

A 12% standard solution of lignocaine benzyl benzoate in methanol was prepared. 0.1ml was pipetted into a 150ml clear, flat bottle, and the methanol allowed to evaporate. A 5% standard solution of the surfactant in white spirit was prepared and 24ml transferred to the test bottle. The surfactant and lignocaine benzyl benzoate were well mixed and 126ml white spirit added to bring the total volume to 150ml. This solution was then shaken vigorously.

EXAMPLE 15

80 ppm Lignocaine benzyl benzoate/0.4% Aliquat 336

A 12% standard solution of lignocaine benzyl benzoate in methanol was prepared. 0.1ml was pipetted into a 150ml clear, flat bottle, and the methanol allowed to evaporate. A 5% standard solution of the surfactant in white spirit was prepared and 12ml transferred to the test bottle. The surfactant and lignocaine benzyl benzoate were well mixed and 138ml white spirit added to bring the total volume to 150ml. This solution was then shaken vigorously.

EXAMPLE 16

50 ppm Lignocaine benzyl benzoate/0.5% Cetyl pyridinium chloride

A 7.5% standard solution of lignocaine benzyl benzoate in methanol was prepared. 0.1ml was pipetted into a 150ml clear, flat bottle, and the methanol allowed to evaporate. A 5% standard solution of the surfactant in white spirit was prepared and 15ml transferred to the test bottle. The surfactant and lignocaine benzyl benzoate were well mixed and 135ml white spirit added to bring the total volume to 150ml. This solution was then shaken vigorously.

EXAMPLE 17

100 ppm Lignocaine benzyl benzoate/1.0% Cetyl pyridinium chloride

A 15% standard solution of lignocaine benzyl benzoate in methanol was prepared. 0.1ml was pipetted into a 150ml clear, flat bottle, and the methanol allowed to evaporate. A 5% standard solutiomn of the surfactant in white spirit was prepared and 30ml transferred to the test bottle. The surfactant and lignocaine benzyl benzoate were well mixed and 120ml white spirit added to bring the total volume to 150ml. This solution was then shaken vigorously.

EXAMPLE 18

30 ppm Lignocaine benzyl benzoate/0.2% Aliquat 336

A 4.5% standard solution of lignocaine benzyl benzoate in methanol was prepared. 0.1ml was pipetted into a 150ml clear, flat bottle, and the methanol allowed to evaporate. A 5% standard solution of the surfactant in white spirit was prepared and 6ml transferred to the test bottle. The surfactant and lignocaine benzyl benzoate were well mixed and 144ml white spirit added to bring the total volume to 150ml. This solution was then shaken vigorously.

EXAMPLE 19

40 ppm Lignocaine benzyl benzoate/0.2% Aliquat 336

A 6.0% standard solution of lignocaine benzyl benzoate in methanol was prepared. 0.1ml was pipetted into a 150ml clear, flat bottle, and the methanol allowed to evaporate. A 5% standard solution of the surfactant in white spirit was prepared and 6ml transferred to the test bottle. The surfactant and lignocaine benzyl benzoate were well mixed and 144ml white spirit added to bring the total volume to 150ml. This solution was then shaken vigorously.

EXAMPLE 20

(1) Preparation of a 0.6% lignocaine benzyl benzoate solution in white spirit containing 40% Alimod 2.

3.0g of lignocaine benzyl benzoate was weighed out accurately and stirred with 200g Alimod 2 until completely dissolved. White spirit was then added until a volume of 500ml was reached. Mixture was stirred well.

(2) Preparation of a 30ppm lignocaine benzyl benzoate solution in white spirit containing 0.2% Alimod 2 from above concentrate.

A 5ml aliquot of the solution prepared in Stage (1) was diluted to 100 ml with white spirit and stirred well.

EXAMPLE 21

Using the procedures described in the preceding examples, solutions were prepared having initial concentrations of surfactant and aversive agent according to the following Table. All solutions were clear and stable. Analyses of the levels of aversive agent were undertaken by aqueous extraction and standard HPLC procedures, for example as described in K. Sugden, T. G. Mayne and C. R. Loscombe, in Analyst, 103, pages 653-656 (1978).

The column headed "Final Concentration" gives the result of the aqueous extraction and HPLC analysis and is equivalent to the proportion of aversive agent removed into the aqueous extraction solution.

| Bittering Agent | Surfactant | Surfactant Concentration (% w/v) | Bittering Agent Concentration Initial (ppm) | Bittering Agent Concentration Final (ppm) |
|---|---|---|---|---|
| Lignocaine Benzyl Cyclamate | Aliquat 336 | 0.5 | 50 | 15.3 |
| Lignocaine Benzyl Cyclamate | Aliquat 336 | 1.0 | 50 | 8.5 |
| Lignocaine Benzyl Cyclamate | Aliquat 336 | 0.5 | 100 | 13.4 |
| Lignocaine Benzyl Cyclamate | Aliquat 336 | 1.0 | 100 | 2.8 |
| Lignocaine Benzyl Stearate | Aliquat 336 | 0.5 | 50 | 13 |
| Lignocaine Benzyl Stearate | Aliquat 336 | 1.0 | 50 | 14 |
| Lignocaine Benzyl Stearate | Aliquat 336 | 0.5 | 100 | 24 |
| Lignocaine Benzyl Stearate | Aliquat 336 | 1.0 | 100 | 32 |
| Lignocaine Benzyl Stearate | Synperonic 91/2.5 | 0.5 | 50 | 16 |
| Lignocaine Benzyl Stearate | Synperonic 91/2.5 | 1.0 | 50 | 12 |
| Lignocaine Benzyl Stearate | Synperonic 91/2.5 | 0.5 | 100 | 12 |
| Lignocaine Benzyl Stearate | Synperonic 91/2.5 | 1.0 | 100 | 16 |
| Lignocaine Benzyl Chloride | Aliquat 336 | 0.5 | 50 | 11 |
| Lignocaine Benzyl Chloride | Aliquat 336 | 1.0 | 50 | 13 |
| Lignocaine Benzyl Chloride | Aliquat 336 | 0.5 | 100 | 25 |
| Lignocaine Benzyl Chloride | Aliquat 336 | 1.0 | 100 | 17 |
| Lignocaine Benzyl Chloride | Synperonic 91/2.5 | 0.5 | 50 | 3 |
| Lignocaine Benzyl Chloride | Synperonic 91/2.5 | 1.0 | 50 | 6 |
| Lignocaine Benzyl Chloride | Synperonic 91/2.5 | 0.5 | 100 | 12 |
| Lignocaine Benzyl Chloride | Synperonic 91/2.5 | 1.0 | 100 | 15 |

**Claims**

1. An apolar hydrocarbon-based liquid containing an effective amount of a compound of the general formula

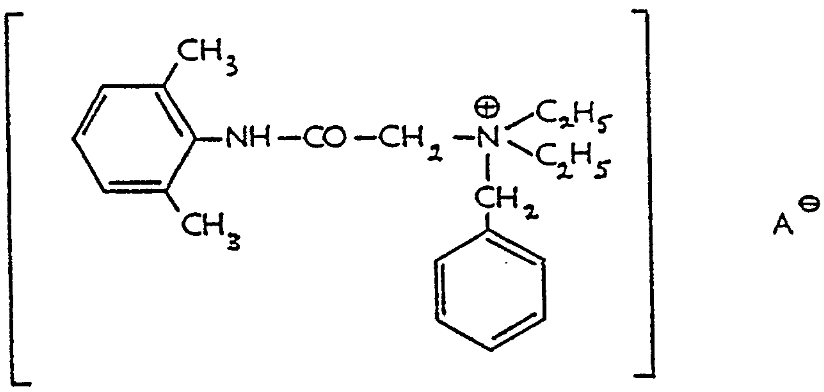

wherein $A^{\ominus}$ is an anion, as an aversive agent, together with a surfactant whereby a stable solution or suspension of said agent is formed.

2. A liquid as claimed in claim 1 wherein the aversive agent is a lignocaine benzyl inorganic acid salt, carboxylate salt, sulphonate salt or saccharide salt.

3. A liquid as claimed in claim 2 wherein the aversive agent is lignocaine benzyl benzoate, lignocaine benzyl chloride, lignocaine benzyl cyclamate, lignocaine benzyl stearate, or lignocaine benzyl saccharide.

4. A liquid as claimed in any preceding claim wherein the concentration of aversive agent is in the range 1 to 5000ppm.

5. A liquid as claimed in any preceding claim wherein the surfactant is an anionic surfactant.

6. A liquid as claimed in any of claims 1 to 4 wherein the surfactant is a non-ionic surfactant.

7. A liquid as claimed in any of claims 1 to 4 wherein the surfactant is a cationic surfactant.

8. A liquid as claimed in claim 7 wherein the surfactant is tricaprylmethylammonium chloride, optionally combined with one or more further surfactants.

9. A liquid as claimed in any preceding claim wherein the concentration of the surfactant is in the range 50ppm to 5% by weight.

10. A composition comprising a compound of the general formula

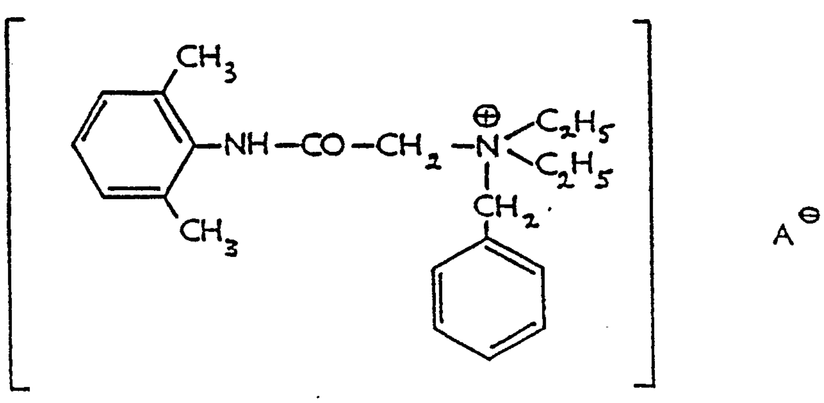

wherein $A^{\ominus}$ is an anion, and a surfactant, said composition forming a stable solution or suspension when dispersed in an apolar hydrocarbon-based liquid.

11. A composition as claimed in claim 10 comprising from 0.5 to 5.0% by weight of aversive agent and from 10 to 99.5% by weight of surfactant.

12. A method of rendering an apolar hydrocarbon-based liquid unpalatable, comprising the addition to said liquid of an effective amount of an aversive agent of the general formula

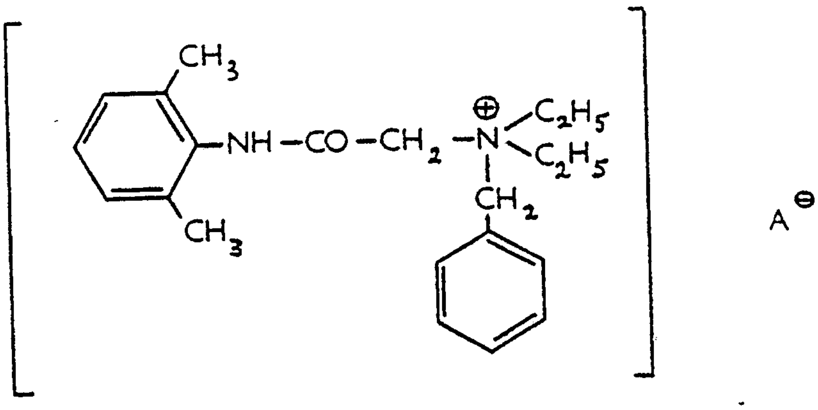

wherein $A^{\ominus}$ is an anion, together with a surfactant, whereby a stable solution or suspension of the said agent is formed.

European Patent Office

# EUROPEAN SEARCH REPORT

Application Number

EP 88 31 1076

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | GB-A- 955 309 (EDINBURGH PHARMACEUTICAL IND. LTD)<br>* Claim; pages 1-4 *<br>--- | 1-5,7,8,10,12 | C 10 L 1/22 |
| Y | CHEMICAL ABSTRACTS, vol. 95, no. 1, 6th July 1981, page 79, abstract no. 692p, Columbus, Ohio, US; T. CHIKARA et al.: "The solubility of local anesthetics in the ionic surfactant micelle solution", & ADV. PHARMACOL. RES. PRACT., PROC. CONGR. HUNG. PHARMACOL. SOC., 3RD 1979 (PUB. 1980). 3(CHEM. STRUCT.-BIOL. ACT. RELAT., QUANT. APPROACHES), 351-5<br>* Abstract *<br>--- | 1-5,7,8,10,12 | |
| A | US-A-2 097 773 (ORELUP)<br>* Whole document *<br>--- | 1-12 | |
| A | EP-A-0 077 552 (MATSUSHITA ELECTRIC INDUSTRIAL CO.,LTD)<br>* Claim; example *<br>--- | 1-12 | TECHNICAL FIELDS SEARCHED (Int. Cl.4) |
| A | US-A-3 147 264 (A.F. KLEIN)<br>* Whole document *<br>--- | 1-12 | C 10 L<br>C 10 M |
| A | US-A-3 935 137 (W. MINKOFF)<br>* Claims; example 1; column 2 *<br>--- | 1-12 | |
| A | US-A-4 064 316 (T.H. CURTIS)<br>* Whole document *<br>--- | 1-12 | |
| A | GB-A- 359 590 (E. VELLNER)<br>* Whole abstract *<br>--- -/- | 1,10,12 | |

The present search report has been drawn up for all claims

| Place of search | Date of completion of the search | Examiner |
|---|---|---|
| THE HAGUE | 01-03-1989 | DE LA MORINERIE B.M.S.B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 88 31 1076

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 105, no. 4, 28th July 1986, abstract no. 29919d, Columbus, Ohio, US; V. HILLGREN et al.: "Mass transfer across liquid-liquid interfaces. VI. Distribution rate of lidocaine in the presence of a non-ionic surfactant", & ACTA PHARM. SUEC. 1986, 23(1), 1-12 --- | 1-12 | |
| A | CHEMICAL ABSTRACTS, vol. 105, no. 11, 15th September 1986, page 55, abstract no. 91133w, Columbus, Ohio, US; S.F. DAVIS et al.: "A preliminary analysis of the suppressive effects of denatonium saccharide" & BULL. PSYCHON. SOC. 1986, 24(3), 229-32 --- | 1-12 | |
| A | CHEMICAL ABSTRACTS, vol. 84, no. 15, 12th April 1976, page 65, abstract no. 99592p, Columbus, Ohio, US; S.M. FERNANDEZ et al.: "Proton magnetic resonance and viscosity studies of the interaction of local anesthetics and mcelles", & ARCH. BIOCHEM:BIOPHYS. 1976, 172(2), 721-5 --- | 1-12 | TECHNICAL FIELDS SEARCHED (Int. Cl.4) |
| A | CHEMICAL ABSTRACTS, vol. 105, no. 22, 1st December 1986, page 386, abstract no. 197096b, Columbus, Ohio, US; A. OMRAY et al.: "Formulation of povidone-iodine aerosol spray", & INDIAN J. PHARM. SCI. 1986, 48(2), 40-2 ----- | 1-12 | |

The present search report has been drawn up for all claims

| Place of search | Date of completion of the search | Examiner |
|---|---|---|
| THE HAGUE | 01-03-1989 | DE LA MORINERIE B.M.S.B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document